# EUROPEAN PATENT APPLICATION

(11) **EP 4 137 177 A1**
(43) Date of publication of application: **22.02.2023**
(21) Application number: 21211393.0
(22) Date of filing: 30.11.2021
(51) Int. Cl.: A61M 5/158

(54) **MANUAL INSERTION AID AND INSERTION AID SYSTEM**

(30) Priority: 18.08.2021 US 202163234317 P
(71) Applicant: F. Hoffmann-La Roche AG, 4070 Basel (CH); Roche Diabetes Care GmbH, 68305 Mannheim (DE)
(72) Inventor: ARNOLD, Marc, 68305 Mannheim (DE); CADIO, Michel Alain Jean, Indianapolis, IN 46250 (US); FREY, Stephan-Michael, 68305 Mannheim (DE); KONYA, Ahmet, 68305 Mannheim (DE); KURZ, Klaus Karl, 68305 Mannheim (DE); SINDLINGER, Stephanie, 68305 Mannheim (DE); WIEGAND, Roland Hans, 68305 Mannheim (DE)
(74) Representative: Pfaffmann, Lucas

(57) **Abstract**

The present invention relates to a manual insertion aid for inserting a cannula unit into a cannula holder. The manual insertion aid comprises a handle and two jaws projecting from the handle and forming a receiving opening that is configured for engaging with a cannula unit. In order to provide a simple and lightweight insertion aid, the minimum clearance between the two jaws is greater than or equal to 1.5 mm and smaller than or equal to 7 mm, and wherein the maximum projecting length of each of the two jaws from the handle is greater than or equal to 4 mm and smaller than or equal to 18 mm.

## Description

The present invention relates to a manual insertion aid for inserting a cannula unit into a cannula holder. The manual insertion aid comprises a handle and two jaws projecting from the handle and forming a receiving opening that is configured for engaging with a cannula unit.

A component of a medical delivery system for delivering a medicament to a patient by infusion is a cannula, which during use is in fluid communication with a reservoir for delivering a medicament out of the reservoir through the cannula into a patient's body. The cannula is configured for piercing the patient's skin. During use, a cannula holder keeps the cannula in its place relative to the patient's body. The cannula holder is configured for being detachably attached to a patient's body and configured for engage the cannula, when the cannula is at least partially inserted into the cannula holder. The cannula is moved towards and into the cannula holder by a force, such that the cannula is at least partially inserted into the cannula holder.

In general, handling and inserting a cannula poses a risk of unintentionally pricking the body with the cannula. Therefore, the cannula is arranged within a casing, which protects the user from being unintentionally injured or pricked, respectively. A further purpose of the casing is to provide a guiding path along which the cannula can be moved for inserting the cannula into the pump holder. In particular, in case the cannula is a soft cannula an insertion needle is moved within the casing together with the cannula, wherein after insertion of the cannula the insertion needle is retracted again.

Insertion can be achieved by using a single use or a resusable inserter device, such as for example described in EP 1 383 560 B1 or WO 2009/001346 A1. Those devices are in particular beneficial for repeatedly exerting a predetermined force to the cannula and insertion needle, each time when inserting the cannula into the cannula holder thereby piercing the patient's skin. Before inserting the cannula into the cannula holder by means of the inserter device, a respective cannula assembly is loaded into the inserting device. The cannula assembly comprises the cannula, and an insertion needle, in case the cannula is a soft cannula. Furthermore, the cannula assembly comprises a casing and a cannula hub.

A disadvantage of using one of the aforementioned inserter devices is that the inserter device has to be carried along by the patient so to be able to insert a cannula into the cannula holder whenever there is a need to replace the cannula holder. Due to its size and weight the inserter device is prone to be forgotten at a certain location, such that a user cannot insert a cannula into the cannula holder by means of the inserter device.

Accordingly, it is an object of the present invention to provide an alternative insertion aid which is reduced in size and costs, and that is, thus, less prone to be forgotten, but can be easily carried along by the patient. It is desirable that the insertion aid can be easily handled by a patient.

At least one of the above objects is solved by a manual insertion aid as outlined above, wherein the minimum clearance between the two jaws is greater than or equal to 1.5 mm and smaller than or equal to 7 mm, and wherein the maximum projecting length of each of the two jaws from the handle is greater than or equal to 4 mm and smaller than or equal to 18 mm.

The two jaws form a receiving opening for engaging part of a cannula hub of a cannula unit through an opening of a casing of a cannula assembly. The two jaws are configured for moving said cannula hub along a predetermined path within the casing in a direction of insertion and a direction of retraction. The direction of retraction is opposite to the direction of insertion. Accordingly, the cannula hub is movable accommodated within the casing of the cannula assembly, wherein the cannula hub at least comprises a cannula that is configured for conveying a fluid into a patient's body. The cannula can be a soft cannula, wherein in this case the cannula unit also comprises an insertion needle that is configured for supporting the soft cannula when penetrating a patient's body. The insertion needle can be distracted from the soft cannula, after the soft cannula has been inserted. The cannula hub is configured for being at least partially attachable to a cannula holder. The cannula hub can comprise a self-sealable septum through which a fluid can be conveyed into the cannula. The cannula hub may further comprises an engagement portion that is configured for being engaged by two jaws of the insertion aid. The cannula hub and engagement portion can be configured that after insertion of the cannula hub into the cannula holder the engagement portion can be detached from the cannula hub thereby leaving the cannula hub inserted into the cannula holder while the engagement portion can be distracted within the casing of the cannula assembly.

According to an embodiment, the minimum clearance between the two jaws is greater than or equal to 2.5 mm and smaller than or equal to 6 mm. The minimum clearance between the two jaws can be greater than or equal to 2.7 mm, and can be smaller than or equal to 5 mm.

In the meaning of the present invention, the term "minimum clearance between the two jaws" refers to the minimum distance between the two jaws within the outermost third of the projecting length allowing an object to be moved in-between the two jaws along the projection direction of the jaws. The minimum clearance between the two jaws is unequal zero, because otherwise the jaws would not form a receiving opening.

According to an embodiment, the two jaws projects parallel to each other away from the handle.

The maximum projecting length of each of the two jaws from the handle can be greater than or equal to 6 mm and smaller than or equal to 15 mm. In an embodiment, the maximum projecting length of each of the two jaws from the handle is greater than or equal to 9 mm and smaller than or equal to 12 mm. In the meaning of the present invention, the maximum projecting length of each of the two jaws determines the length of each jaw measured between its point of connect with the handle along the respective direction of projection and the outermost tip of the respective jaw.

According to an embodiment, each of the two jaws comprises a planar sideward contact surface, the sideward contact surfaces are facing towards each other thereby limiting the clearance between the two jaws. The planar sideward contact surfaces allow an improved load transmission from the manual insertion aid to the cannula hub. The planar sideward contact surfaces, in particular when being parallel to each other, further help to prevent unintended tipping relative to the cannula hub with regard to the axis of projecting of the jaws. The axis of projecting of the jaws is an axis extending in a direction along the direction of projection of the jaws from the handle.

Alternatively or in addition, for allowing an improved load transmission between the manual insertion aid and the cannula hub, according to an embodiment, each of the two jaws comprises a planar lower contact surface, the lower contact surfaces are arranged within a single common imaginary lower plane, and/or each of the two jaws comprises a planar upper contact surface, the upper contact surfaces are arranged within a single common imaginary upper plane. The planar surfaces, in particular in case the cannula hub comprises a correspondingly formed planar contact surfaces, may also prevent an unintended tipping of the manual insertion aid relative to the cannula hub in a rotational direction around the axis of projection of the respective jaw. Planar surfaces may also increase the area of contact between the jaws of the manual insertion aid and the cannula hub of the cannula assembly.

Each of the two jaws can have a maximum height larger than or equal to 1 mm and smaller than or equal to 5 mm, wherein the respective maximum height is measured in a direction perpendicular to the minimum clearance between the two jaws. According to an embodiment, the height of each of the two jaws is larger than or equal to 1.5 mm and smaller than or equal to 3.5 mm. The two jaws can have the same height. According to an embodiment the maximum height of each of the two jaws is 3 mm.

According to an embodiment, the manual insertion aid is mirror symmetrical relative to a first mirror plane extending along the projecting length of the two jaws and along the minimum clearance between the two jaws. The mirror plane virtually divides the manual insertion aid in an upper portion and a lower portion. The mirror symmetry allows an easy handling of the manual insertion aid.

Alternatively, or in addition, according to an embodiment, the manual insertion aid is mirror symmetrical relative to a second mirror plane extending along the projecting length of the two jaws and perpendicular to the minimum clearance between the two jaws. The second mirror plane virtually divides the manual insertion aid in a left hand side portion and a right hand side portion, and allows an easy handling of the manual insertion aid.

In order for improving the handling of the manual insertion aid, and, in particular, for supporting a user exerting a load to the cannula hub by means of the manual insertion aid more carefully, according to an embodiment, the handle comprises a pattern configured for increasing the slip resistance, preferably the pattern comprises repetitive projections and/or depressions. For instance, a non-exhaustive selection of patterns comprises ribs, truncated pyramids, calottes, depressions, recesses, etc.

According to an embodiment, each of the two jaws has a free front tip with a chamfer. The chamfer simply entering the casing of the cannula assembly with the jaws and thereby helps to engage the jaws with the cannula hub. For instance, the chamfer is composed of a surface angled relative to the axis of projection of the jaws from the handle.

The two jaws can be angled relative to a plane extending perpendicular to the thickness of the handle, wherein the thickness of the handle is smaller than the length of the handle and smaller than or equal to the width of the handle. In the meaning of the present invention "the length of the handle" correspond to the extension of the handle starting from the point of contact with the jaw to the opposing end of the handle. The angle formed between the jaws and the plane extending perpendicular to the thickness of the handle is, according to an embodiment, smaller than or equal to 60° and larger than or equal to 20°.

According to an embodiment, the manual insertion aid comprises a connector portion configured for attaching the manual insertion aid to a key ring or zipper. Attaching the manual insertion aid to a key ring or zipper helps to carry the manual insertion aid along, such that the manual insertion aid is less prone to be forgotten or lost. According to a non-exhaustive example, the manual insertion aid, preferably the handle of the manual insertion aid, can comprise a through-hole to thread a key ring or zipper. In another embodiment, the handle of manual insertion aid itself also forms a handle of a zipper.

According to an embodiment, the handle forms at least part of token or comprises a compartment that is configured for temporarily holding a token. A manual insertion aid comprising a handle that forms at least part of a token cannot only be used for manual inserting a cannula hub into a cannula holder, but can also be used for borrowing an object, e.g. a shopping cart. However, instead of forming at least part of a token, the handle may comprise a compartment for temporarily receiving a token. Accordingly, the compartment of the handle is correspondingly formed to receive a token. For instance, the handle can have a form of a coin, and the compartment can be a disk-like recess or a clamp for receiving a coin-like formed token. According to an embodiment, the radius of the coin-shaped handle can be greater than or equal to 10 mm and smaller than or equal to 15 mm.

The purpose of the manual insertion aid can be further increased when the handle comprises a filing aid portion that is configured for engaging a piston rod connected to a plunger that is moveable inserted into an insulin reservoir. A medicament delivery device, such an insulin pump, comprises a cartridge with a reservoir. The reservoir can be filled with a medicament, e.g. insulin, wherein the medicament can be withdrawn from the reservoir by actuating a piston rod so to move a plunger within in the reservoir. For drawing up the reservoir with the medicament, e.g. insulin, the plunger can be retracted by means of the manual insertion aid connected to the piston rod with help of the filing aid portion. For example, the filing aid portion comprises a receiving portion that is configured for receiving a coupling element of the piston rod, such as for example a drive nut. The filing aid portion and the two jaws can be arranged on opposing sides of the handle of the manual insertion aid.

According to an embodiment, the handle comprises an at least partially circular outer edge, wherein the at least partially circular edge of the handle is part of an imaginary circle encircling the outer free tips of the jaws. According to an embodiment the at least partially circular edge of the handle and the free tips of the two jaws are lying on a common imaginary circle. By ending on an imaginary circle or by being encircled by an imaginary circle which is at least partially congruent with the circular edge of the handle allows a compact manual insertion aid without parts extensively protruding beyond the handle.

The present invention further relates to a cannula assembly insertion system that solves at least one of the objections mentioned in the beginning, wherein the cannula assembly insertion system comprises a manual insertion aid as described above and a cannula assembly, wherein the cannula assembly comprises a casing accommodating a cannula hub, the cannula hub is movable within the casing along a predetermined path and the cannula hub comprises a cannula and an engagement portion, wherein the engagement portion is configured for being temporarily engaged by the jaws of the manual insertion aid so to move the cannula hub along the predetermined path.

According to an embodiment of the insertion aid system, the cannula hub further comprises an insertion needle and a soft cannula, wherein the insertion needle is attached to the engagement portion, the engagement portion is releasably attached to the cannula hub when moving the cannula hub by the jaws of the manual insertion aid in a direction of insertion the engagement portion, and the engagement portion is detached from the cannula hub when moving the engagement portion together with the insertion needle by the jaws of the manual insertion aid in a direction of retraction. The direction of retraction is an opposite direction to the direction of insertion.

According to an embodiment, the engagement portion of the cannula assembly is formed by a cylinder and two parallel flanges, wherein each of the flanges forming a circumferential overlap radially protruding outwards from the outer circumferential wall of the cylinder, the outer diameter of the outer circumferential wall of the cylinder is smaller than or equal to the minimum clearance between the two jaws of the manual insertion aid. The distance between the two flanges of the engagement portion along the longitudinal cylinder axis is larger than the maximum height of the jaws of the manual insertion aid.

According to an embodiment the maximum projecting length of each of the two jaws from the handle is at least half of the outer diameter of the circumferential overlap protruding outwards from the outer circumferential wall of the cylinder.

Insofar as in the foregoing as well as in the following detailed description of embodiments and claims, features of the manual insertion aid described in the context of the insertion aid system are applicable to the manual insertion aid on its own, i.e. irrespective of the presence of a cannula assembly. Likewise, insofar as in the foregoing as well as in the following detailed description of embodiments and claims, features of the cannula assembly are described those are applicable to the Insertion aid system, irrespective of the presence of a manual insertion aid.

Further advantages, features and technical effects of the present invention are apparent from the following description of embodiments and the accompanying figures.
- Figure 1: shows a schematic view of a manual insertion aid according to an embodiment of the present invention;
- Figure 2: shows a schematic view of a manual insertion aid according to another embodiment of the present invention;
- Figure 3: shows a schematic view of a manual insertion aid according to another embodiment of the present invention;
- Figure 4: shows a schematic view of insertion aid system according to an embodiment of the present invention; and
- Figure 5: shows a schematic cross-sectional view of the insertion aid system according to figure 4.

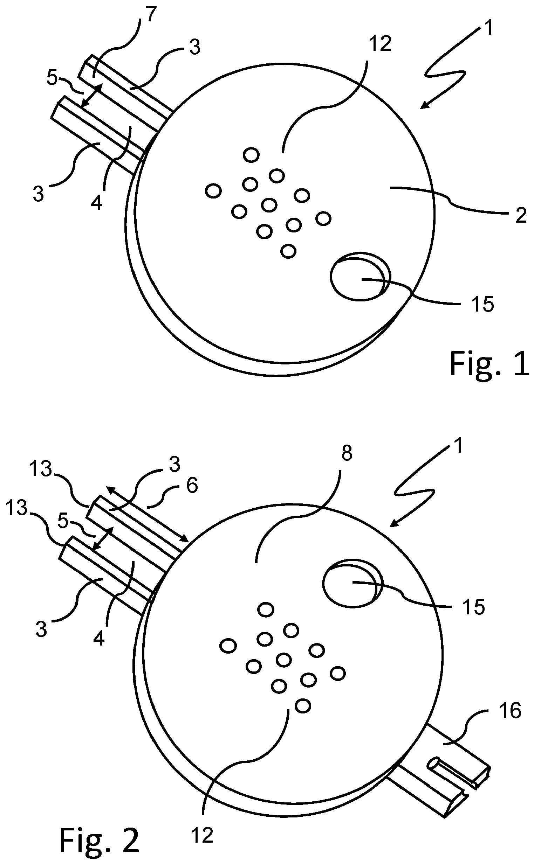

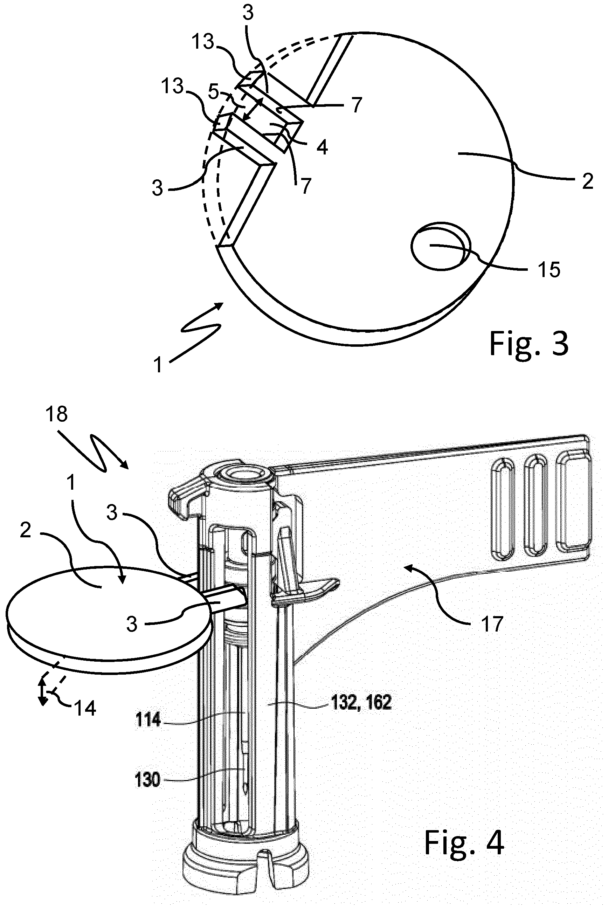

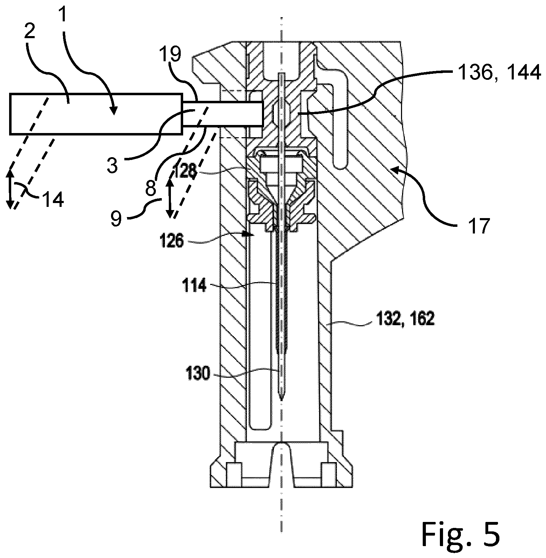

Figure 1 shows a manual insertion aid 1 for inserting a cannula unit into a cannula holder according to an embodiment of the present invention. The manual insertion aid 1 comprises a handle 2 allowing a user handling the manual insertion aid 1. The handle 2 has disk-like shape and comprises two jaws 3 projecting from the handle 1. The two jaws 3 form a receiving opening 4 that is configured for engaging with a cannula unit as it is for example shown in figures 4 and 5. The jaws 3 are projecting parallel to each other from the handle 2, wherein the minimum clearance 5 between the two jaws 3 within the outermost third of the projecting length of the jaws 3 is greater than or equal to 1.5 mm and smaller than or equal to 7 mm. The maximum projecting length 6 of each of the jaws 3 from the handle, i.e. the maximum length of each jaw 3 from the handle 2 to the outermost tip of the jaw, is greater than or equal to 4 mm and smaller than or equal to 18 mm. During use, as for example shown in figures 4 and 5 with regards to another embodiment, the two jaws 2 partially encompassing a respective portion of the cannula unit.

For easy handling the handle 2 comprises a pattern 12 that is configured for increasing the slip resistance. As shown for example in figures 1 and 2, pattern 12 comprises repetitively arranged projections that are projecting from the handle 2.

The manual insertion aid 1 further comprises a connector portion 15 that is configured for attaching the manual insertion aid 1 to a key ring or a zipper. Here, the connector portion is a through hole in the handle 2 that can be engaged by a key ring or a zipper.

A second embodiment of a manual insertion aid according to the present invention is shown in figure 2. The manual insertion aid according to figure 2 is similar to the manual insertion aid according to figure 1, and differs only in the location of the connector portion 15 and in having an additional filing aid 16. The filing aid 16 is configured for engaging a piston rod that is moveable inserted into an insulin reservoir. Therefore, the filing aid 16 comprise a receiving portion that is configured for receiving a coupling element of a piston rod, such as for example a drive nut.

Figure 3 shows another embodiment of a manual insertion aid 1 comprising a handle 2 and two jaws 3. The two jaws project from the handle 2 thereby forming a receiving opening 4 that is configured for engaging with a cannula unit. Each of the two jaws 3 comprise a planar lower contact surface 8, as for example indicated in figure 5, and a planar upper contact surface 19, as for example indicated in figure 5 as well. The planar lower contact surfaces 8 are in contact with a respective contact surface of a cannula unit when the manual insertion aid 1 engages a cannula unit and is moving the cannula unit towards a cannula holder. Likewise, the upper contact surfaces 19 are in contact with a respective contact surface of a cannula unit when the manual insertion aid 1 engages a cannula unit and is moving the cannula in a direction away from the cannula holder.

Furthermore, each of the two jaws 3 comprise a planar sideward contact surface 7. The contact surfaces 7 of the two jaws 3 are facing towards each other thereby delimiting the receiving opening 4. The distance between the planar sideward contact surfaces 7 corresponds to the minimum clearance 5 between the two jaws and, thus, is greater than or equal to 1.5 mm and smaller than or equal to 7 mm. The sideward planar contact surfaces 7 cannot only be used for engaging the cannula unit, but can also be used as a guiding element which when they are in contact with respective surfaces of a cannula assembly define a predefined guiding path for inserting the cannula unit into a cannula holder.

Each of the two jaws comprise an outer free tip 13. The free tips 13 can comprise a chamfer which ease engaging with a cannula unit.

The handle 2 of the manual insertion aid as shown in figure 2 further comprises a partially circular outer edge. The partially circular edge of the handle is part of an imaginary circle encircling the outer free front tips 13 of the jaws 3. I.e. the front tips 13 of the jaws 3 are ending on both ending on the same imaginary circle partially overlapping with the partially circular outer edge of the handle 2.

The manual insertion aid 1 comprises a connector portion 15 that is configured for attaching the manual insertion aid to a key ring or a zipper. The manual aid 1 is mirror symmetrical relative to a first mirror plane extending along the projecting length of the two jaws 3 and along the minimum clearance between the two jaws 3. I.e. the first mirror plane virtually divides the manual insertion aid 1 into two mirror symmetrical upper and lower portions. Furthermore, the manual insertion aid 1 is mirror symmetrical relative to a second mirror plane extending along the projecting length of the two jaws 3 and perpendicular to the minimum clearance between the two jaws 3.

Figures 4 and 5 show a manual insertion aid 1 engaging a cannula unit of a cannula assembly 17. Together the manual insertion aid 1 and the cannula assembly 17 form an insertion aid system 18. The cannula assembly 17 comprises a casing 162 having a protective element 132 that accommodate a cannula unit with a cannula hub 128. Initially, the cannula hub 128 comprises a soft cannula 114 and an insertion needle 130. The cannula hub 128 further comprises an engagement element 136 for engaging with the two jaws 3. The engagement element 136 comprises a cylindrical portion 144 and two overlapping portions which overlap the cylindrical portion so to form a ring-like recess for engaging with the jaws 3. The protective element 132 comprise two slots. During use, the jaws 3 can reach through the slots into the casing 162 so to engage with the engagement element 136 of the cannula hub.

The manual insertion aid 1 of the insertion aid system 18 can be configured, for example, as any one of the manual insertion aids 1 as shown in figures 1 to 3. Handle 2 of the manual insertion aid 1 has a thickness 14 which is larger than or equal to the maximum height 9 of the jaws 3.

### List of reference numbers

- 1: manual insertion aid
- 2: handle
- 3: jaw
- 4: receiving opening
- 5: minimum clearance between the two jaws of the handle
- 6: maximum projecting length of jaw
- 7: planar sideward contact surface
- 8: planar lower contact surface
- 9: maximum height of jaw
- 10: first mirror plane
- 11: second mirror plane
- 12: pattern
- 13: free front tip
- 14: thickness of handle
- 15: connector portion
- 16: filing aid portion
- 17: cannula assembly
- 18: insertion aid system
- 19: planar upper contact surface

## Claims

1. Manual insertion aid (1) comprising:
• a handle (2) and
• two jaws (3) projecting from the handle (2) and forming a receiving opening (4) that is configured for engaging with a cannula unit,
• wherein the minimum clearance (5) between the two jaws (3) is greater than or equal to 1.5 mm and smaller than or equal to 7 mm,
• wherein the maximum projecting length (6) of each of the two jaws (3) from the handle (2) is greater than or equal to 4 mm and smaller than or equal to 18 mm.

2. Manual insertion aid (1) according to claim 1, wherein each of the two jaws (3) comprises a planar sideward contact surface (7), the sideward contact surfaces (7) are facing towards each other thereby delimiting the minimum clearance (5) between the two jaws (3).

3. Manual insertion aid (1) according to claim 1 or 2, wherein each of the two jaws (3) comprises a planar lower contact surface (8), the lower contact surfaces (8) are arranged within a single common imaginary lower plane, and/or each of the two jaws (3) comprises a planar upper contact surface (19), the upper contact surfaces (19) are arranged within a single common imaginary upper plane.

4. Manual insertion aid (1) according to any one claims 1 to 3, wherein each of the two jaws (3) has a maximum height larger (9) than or equal to 1 mm and smaller than or equal to 5 mm , wherein the respective maximum height (9) is measured in a direction perpendicular to the minimum clearance (5) between the two jaws (3).

5. Manual insertion aid (1) according to any one of claims 1 to 4, wherein the manual insertion aid (1) is mirror symmetrical relative to a first mirror plane extending along the maximum projecting length (6) of the two jaws (3) and along the minimum clearance (5) between the two jaws (3).

6. Manual insertion aid (1) according to any one of claims 1 to 5, wherein the manual insertion aid (1) is mirror symmetrical relative to a second mirror plane extending along the maximum projecting length (6) of the two jaws (3) and perpendicular to the minimum clearance (5) between the two jaws (3).

7. Manual insertion aid (1) according to any one of claims 1 to 6, wherein the handle (2) comprises a pattern (12) configured for increasing the slip resistance, preferably the pattern comprises repetitive projections and/or depressions.

8. Manual insertion aid (1) according to any one of claims 1 to 7, wherein each of the two jaws (3) has a free front tip with a chamfer.

9. Manual insertion aid (1) according to any one of claims 1 to 8, wherein each of the two jaws (3) are angled relative to a plane extending perpendicular to the thickness (14) of the handle (2), wherein the thickness (14) of the handle (2) is smaller than the length of the handle (2) and smaller than or equal to the width of the handle (2).

10. Manual insertion aid (1) according to any one of claims 1 to 9, wherein the manual insertion aid (1) comprises a connector portion (15) configured for attaching the manual insertion aid (1) to a key ring or zipper.

11. Manual insertion aid (1) according to any one of claims 1 to 10, wherein the handle (2) comprises a compartment that is configured for temporarily holding a token.

12. Manual insertion aid (1) according to any one of claims 1 to 11, wherein the handle (2) comprises a filing aid portion (16) that is configured for engaging a piston rod that is moveable inserted into an insulin reservoir.

13. Manual insertion aid (1) according to any one of claims 1 to 12, wherein the handle (2) comprises an at least partially circular outer edge, wherein the at least partially circular edge and the free front tips (13) of the two jaws (3) are forming an imaginary circle.

14. Insertion aid system (18) comprising a manual insertion aid (1) according to any one of claims 1 to 13, and a cannula assembly, wherein the cannula assembly (17) comprises a casing accommodating a cannula hub, wherein the cannula hub is movable within the casing along a predetermined path and the cannula hub comprises a cannula and an engagement portion, wherein the engagement portion is configured for being temporarily engaged by the jaws of the manual insertion aid so to move the cannula hub along the predetermined path.
